# EUROPEAN PATENT APPLICATION

(11) **EP 3 998 065 A1**
(43) Date of publication of application: **18.05.2022**
(21) Application number: 20840297.4
(22) Date of filing: 13.07.2020
(51) Int. Cl.: A61K 9/16, A61K 9/00, A61K 31/27, A61P 25/28

(54) **LONG-LASTING FORMULATION CONTAINING RIVASTIGMINE, AND METHOD FOR PREPARING SAME**

(30) Priority: 12.07.2019 KR 20190084775
(71) Applicant: G2Gbio, Inc., Daejeon 34054 (KR)
(72) Inventor: LEE, Heeyong, Daejeon 34032 (KR); SEOL, Eunyoung, Daejeon 34080 (KR); LEE, Juhan, Daejeon 35218 (KR); LEE, Yeonkyeong, Daejeon 34046 (KR); PARK, Donghyun, Daejeon 34165 (KR); CHOE, Heekyoung, Ansan-si, Gyeonggi-do 15521 (KR)
(74) Representative: Ricker, Mathias
(86) International application number: PCT/KR2020/009221
(87) International publication number: WO 2021/010719

(57) **Abstract**

The present invention relates to a sustained release microsphere for long-lasting injectable formulations comprising one or more active ingredients selected from the group consisting of rivastigmine and pharmaceutically acceptable poorly soluble salts thereof and a biodegradable polymer, and a long-lasting injectable formulation for preventing or treating Alzheimer's disease comprising the same and a method for preparing the microsphere, and it can reduce side effects of the patient's gastrointestinal tract, which are frequently seen in conventional oral administration agents, and increase the adaptability of taking medicine, thereby maximizing the therapeutic effect, by providing a long-lasting injectable formulation comprising a rivastigmine sustained release microsphere, which has a high content while effectively controlling the initial burst drug release.

## Description

### [TECHNICAL FIELD]

The present invention relates to a long-lasting composition for preventing or treating Alzheimer's disease comprising rivastigmine and a method for preparing the same, and more specifically, it relates to a sustained release microsphere for injectable formulations that can minimize problems caused by rapid initial release by effectively controlling the initial release of rivastigmine, while exhibiting a long duration of drug efficacy, by comprising a biodegradable polymer carrier and high content rivastigmine, and a long-lasting therapeutic agent for Alzheimer's disease comprising this sustained release microsphere, and a method for preparing the same.

### [BACKGROUND ART]

Recently, as the number of dementia patients rapidly increases due to life extension and an increase in the elderly population, the management of dementia patients is emerging as a serious social problem. Dementia refers to a syndrome characterized by complex cognitive impairment characterized by memory loss, intellectual regression, personality change and behavioral abnormalities. This symptom is a degenerative disease related to brain, which is the central nervous system, and irreversible dysfunction of the neural network is caused by the slow death of nerve cells that eventually cause permanent loss of the function of the human body. The cause of dementia has not yet been clearly elucidated, and since it has various etiological and pathophysiological factors, there is no therapeutic agent that can fundamentally treat dementia. Most of Alzheimer's dementia therapeutic agents currently used as indirect treatment methods are inhibitors of acetylcholinesterase, an acetylcholine degrading enzyme, and donepezil (trade name: Aricept), tacrine (trade name: Cognex), rivastigmine (trade name: Exelon), galantamine (trade name: Reminyl), and the like belong thereto. Rivastigmine is an acetylcholinesterase (AChE) inhibitor and is widely used in treatment of mild to moderate Alzheimer's disease.

The rivastigmine formulation currently used commercially is prescribed to Alzheimer's disease patients in the form of an oral tablet twice a day or a patch form once a day. However, in general, the oral rivastigmine formulation has poor drug adherence, and the short half-life of rivastigmine in blood results in large fluctuations between the highest and lowest concentrations, resulting in nausea, vomiting, abdominal pain, diarrhea, indigestion, loss of appetite, dizziness, agitation, depression, headache, insomnia, confusion, paralysis, sweating, tremor, malaise, upper respiratory and reproductive system infection, and the like are known to occur. In addition, it is not easy to administer drugs orally to patients with advanced dementia.

Rivastigmine, currently used as a transdermal patch, is administered at a dose of 4.6 mg to 13.3 mg once a day. Rivastigmine transdermal patch has relatively few gastrointestinal side effects compared to oral drugs, but has side effects such as skin irritation, and the like, and has various technical problems such as reduction of adhesion, non-uniformity of skin permeation rate, and the like. Therefore, a study to develop a sustained release injectable agent containing rivastigmine using a biodegradable polymer has been proposed.

In Chinese patent CN101708164A, a microsphere containing rivastigmine tartrate was prepared and evaluated using a copolymer of lactide and glycolide or polylactide, which is a biodegradable polymer. However, the patent did not disclose the in vivo test result of the rivastigmine microsphere, and the in vitro release result showed that more than 80% of the rivastigmine was released for one to two weeks, and therefore, there is a problem that it is not suitable as a formulation showing long-term medicinal effect. In addition, the amount of rivastigmine in the microsphere is very low as up to 9.11% by weight as rivastigmine tartrate and 5.69 % by weight as rivastigmine, so there is a difficulty in administering a very large amount of microsphere when administered to a patient.

### [DISCLOSURE]

### [TECHNICAL PROBLEM]

The present invention is designed to solve the above problems of the conventional rivastigmine agents, and an object of the present invention is to provide a sustained release microsphere for injective formulations containing rivastigmine, which has a high drug content of rivastigmine in the microsphere and has a characteristic of stable drug release for a long period of time without initial burst release, and has excellent injectability and uniform particle size distribution, a sustained release injection formulation for preventing or treating Alzheimer's disease comprising the microsphere, and a method for preparing the microsphere.

### [TECHNICAL SOLUTION]

The present invention provides a sustained release microsphere for injectable formulations comprising a poorly soluble active ingredient and a biodegradable polymer, wherein the active ingredient is one or more of active ingredients selected from the group consisting of rivastigmine and pharmaceutically acceptable poorly soluble salts thereof and an amount of the active ingredient is comprised in an amount of 7 % by weight or more as rivastigmine based on the total microsphere and it has excellent injectability and the uniform particle size, and a sustained release injectable formulation for preventing or treating Alzheimer's disease comprising the microsphere and a method for preparing the same.

### [ADVANTAGEOUS EFFECTS]

The sustained release microsphere for injectable formulations containing rivastigmine according to the present invention comprises rivastigmine in a high content and has the excellent injectability and uniform particle size, different from conventional microspheres containing rivastigmine, and thus, rivastigmine injectable formulations for long-term administration can be prepared, and also the release of rivastigmine is effectively controlled to prevent the initial burst rivastigmine release. Accordingly, in the sustained release injectable formulation comprising the microsphere, rivastigmine can be maintained at an effective concentration in blood of dementia patients for a long period of 1 week or more to 3 months or more with a single administration, thereby increasing the medication compliance of dementia patients, as well as minimizing side effects to maximize the therapeutic effects.

### [BRIEF DESSCRIPTION OF THE DRAWINGS]

FIG. 1a, FIG. 1b and FIG. 1c are scanning electron microscope photographs of rivastigmine-containing microspheres obtained according to Sample 2-3, Sample 3-3 and Sample 3-4, respectively.
FIG. 2 is a graph showing the result of measuring the *in vitro* drug release rate of rivastigmine-containing microspheres obtained according to Sample 2-3, Sample 3-3 and Sample 3-4 by time (day 0 - day 49).
FIG. 3a is a graph showing the pharmacokinetic result measured by time after a single intramuscular administration of the microsphere obtained according to Sample 3-7 to rats.
FIG. 3b is a graph showing the pharmacokinetic result measured by time after a single intramuscular administration of the microsphere obtained according to Sample 3-8 to rats.
FIG. 3c is a graph showing the pharmacokinetic result measured by time after a single intramuscular administration of the microsphere obtained according to Sample 3-9 to rats.

### [BEST MODE]

The sustained release microsphere for injectable formulations of the present invention comprises one or more selected from the group consisting of a poorly soluble drug and poorly soluble salt thereof as an active ingredient, specifically, one or more selected from the group consisting of rivastigmine and pharmaceutically acceptable poorly soluble salt thereof, in which an amount of the active ingredient in the microsphere is comprised in an amount of 7 % by weight or more, 9 % by weight or more, or 10 % by weight or more, 35 % by weight or less or 30 % by weight or less as rivastigmine based on the total microsphere weight. The present invention is characterized by comprising poorly soluble rivastigmine or a poorly soluble salt of rivastigmine as an active ingredient, different from a microsphere containing rivastigmine comprising water-soluble rivastigmine tartrate.

The rivastigmine, specifically, rivastigmine freebase, which may be comprised as an active ingredient, is a liquid phase at a room temperature, and is a poorly soluble drug with a solubility in water of 5 mg/mL or less.

In the present invention, the pharmaceutically acceptable poorly soluble salt of rivastigmine means that when it is formed as an organic acid addition salt, the formed salt has a solubility in water of 10 mg/mL or less at a room temperature, and for example, it may be one or more poorly soluble salts selected from the group consisting of xinafoate, napadisilate and pamoate. Preferably, it may be rivastigmine pamoate. The organic acid part (namely, xinafoic acid, napadisilate acid and pamoic acid) of the poorly soluble salt can allow for better control of the release of rivastigmine while increasing the encapsulation efficiency of rivastigmine in the microsphere according to the present invention.

When rivastigmine pamoate is comprised in the microsphere of the present invention as an active ingredient, the molar ratio of rivastigmine and pamoic acid in the rivastigmine pamoate may be 1:0.3 to 1:1, 1:0.3 to 1:0.8 or 1:0.4 to 1:0.7, but not limited thereto. The molar ratio of rivastigmine and pamoic acid in the above range may be more preferable in terms of increasing the encapsulation efficiency of rivastigmine and better controlling the release of the drug.

In the sustained release microsphere for injectable formulations according to the present invention, when a pharmaceutically acceptable poorly soluble salt of rivastigmine, for example, rivastigmine pamoate is encapsulated in the microsphere, a microsphere containing rivastigmine in a high content, in which the content as rivastigmine is 7 % by weight or more, 9 % by weight or more, or 10 % by weight or more based on the total microsphere weight may be prepared. In contrast, when rivastigmine or rivastigmine tartrate is used in conventional rivastigmine-containing microsphere formulations, rivastigmine cannot be comprised in a high content in the microsphere, for example, 7 % by weight or more, 9 % by weight or more, or 10 % by weight, and therefore, there was a problem that it was difficult to develop a long-lasting formulation, but the sustained release microsphere for injectable formulations according to the present invention solve the problems of the conventional rivastigmine-containing microsphere formulations.

The sustained release microsphere for injectable formulations containing rivastigmine of the present invention may further comprise fatty acid having 4 or more carbons in the carbon chain or pamoic acid as a release adjusting agent. The examples of the fatty acid used as the release adjusting agent may include one or more selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, isocrotonic acid, oleic acid, elaidic acid, sorbic acid, linoleic acid and arachidonic acid.

Preferably, the content of the organic acid (for example, xinafoic acid, napadisilate acid and pamoic acid) part in the rivastigmine poorly soluble salt comprised in the sustained release microsphere for injectable formulations according to the present invention the fatty acid or pamoic acid used as a release adjusting agent may be 2.0 to 50 % by weight based on the total microsphere weight. For example, when the sustained release microsphere for injectable formulations according to the present invention does not comprise fatty acid or pamoic acid as a release adjusting agent, the content of the organic acid part in the rivastigmine poorly soluble salt may be 2.0 to 50 % by weight based on the total microsphere weight. In addition, when the sustained release microsphere for injectable formulations according to the present invention comprises rivastigmine freebase as an active ingredient and comprises fatty acid or pamoic acid as a release adjusting agent, the sum of the content of fatty acid or pamoic acid comprised as a release adjusting agent in the microsphere may be 2.0 to 50 % by weight based on the total microsphere weight. Furthermore, when the sustained release microsphere for injectable formulations according to the present invention comprises a rivastigmine poorly soluble salt as an active ingredient and comprises fatty acid or pamoic acid as a release adjusting agent, the sum of the contents of the organic acid part in the rivastigmine poorly soluble salt in the microsphere, and the fatty acid or pamoic acid comprised as a release adjusting agent may be 2.0 to 50 % by weight based on the total microsphere weight.

The sustained release microsphere for injectable formulations comprises a biodegradable polymer together with the active ingredient, and for example, the sustained release microsphere for injectable formulations is prepared using a biodegradable polymer with intrinsic viscosity of 0.16-1.9 dL/g. The intrinsic viscosity of the biodegradable polymer used in the present invention refers to one measured in chloroform at 25°C at a concentration of 0.1 % (w/v) using Ubbelohde viscometer. When the intrinsic viscosity of the biodegradable polymer is 0.16 dL/g or more, the molecular weight of the polymer is sufficient and the sustained release effect of the rivastigmine drug may be further improved, and when the intrinsic viscosity is 1.9 dL/g or less, the release of the rivastigmine drug is not delayed too much and an appropriate effect may be exhibited. In addition, when a polymer satisfying the above intrinsic viscosity range is used, a more reproducible microsphere without the problem of having to use an excessive amount of a preparation solvent due to high viscosity of the polymer during microsphere preparation, which may occur when a polymer having high intrinsic viscosity is used.

The biodegradable polymer used in the present invention may have a weight-average molecular weight of 4,000 to 240,000. For example, the weight-average molecular weight of the biodegradable polymer includes all sub-numeric ranges within the above range, such as a weight-average molecular weight of 4,000 to 100,000, a weight-average molecular weight of 7,000 to 50,000, a weight-average molecular weight of 5,000 to 20,000, a weight-average molecular weight of 10,000 to 18,000 and a weight-average molecular weight of 18,000 to 28,000.

The example of the biodegradable polymer may be one or more polymers selected from the group consisting of poly(lactide-co-glycolide), poly(lactide-co-glycolide)glucose, polylactide, polyglycolide, polycaprolactone or mixture thereof; polyglycolide, polylactide and a copolymer of polyglycolide and polylactide, and preferably, it may be poly(lactide-co-glycolide) or polylactide. In one preferable aspect, in case of the polyglycolide and polylactide copolymer, the molar ratio of the lactide to glycolide in the copolymer may be 40:60 to 90:10, 45:55 to 85:15 or 50:50 to 75:25, for example, 45:55, 50:50, 75:25, or 85:15.

When the biodegradable polymer is comprised in 2 or more kinds, it may be a combination or blend of polymers of different kinds of the exemplified polymers, but it may be the same kind of polymers having different intrinsic viscosity and/or monomer ratios (for example, a combination or blend of two or more of poly(lactide-co-glycolide) having different intrinsic viscosity), or the same kind of polymers having different end groups (for example, the end group is ester or end group is acid). The example of commercially available biodegradable polymer, which may be used in the present invention, may include RG 502H, RG 503H, RG 504H, RG 502, RG 503, RG 504, RG 653H, RG 752H, RG 753H, RG 752S, RG 755S, RG 756S, RG 858S, R 202H, R 203H, R 205H, R 202S, R 203S, R 205S, which is Resomer-based of Evonik Rohm GmbH, and PDL 02A, PDL 02, PDL 04, PDL 05, PDLG 7502A, PDLG 7502, PDLG 7507, PDLG 5002A, PDLG 5002, PDLG 5004A, PDLG 5004, PDLG 5010, PL 10, PL 18, PL 24, PL 32, PL 38, PDL 20, PDL 45, PC 02, PC 04, PC 12, PC 17, PC 24 of Corbion alone, or in combination or blended, but not limited thereto. In one embodiment, to prepare the microsphere according to the present invention, a microsphere was prepared using Resomer R 203H, R 205S, RG 753H and RG 858S alone or in combination or blended. A suitable molecular weight or blending ratio of the biodegradable polymer may be appropriately selected by those skilled in the art in consideration of the decomposition rate of the biodegradable polymer and the resulting drug release rate, and the like.

Based on the total weight of the sustained release microsphere for injectable formulations according to the present invention, the rivastigmine content may be 7 % by weight or more, 9 % by weight or more, or preferably, 10 % by weight or more, based on rivastigmine freebase. When the content of rivastigmine in the microsphere is 9 % by weight or more, the content of rivastigmine comprised in the single microsphere is high, and thus even a relatively small amount of microsphere can represent a sufficient long-term drug release, and the single dose required for long-term drug release is reduced, so it has convenience in administration, low side effects and excellent therapeutic effect. In particular, in that it is advantageous that the administration interval is long, since Alzheimer's disease or Alzheimer's dementia, which is target disease of rivastigmine, requires a very long-term drug administration, and in most cases, it is difficult to administer the drug by itself, the small dose, long-lasting effect, long administration interval and administration convenience of the present invention are particularly advantageous for the use of target disease of rivastigmine.

The higher the content of rivastigmine encapsulated in the individual microsphere, the more preferable because the dosage of the injection formulation containing the microsphere decreases, but in general, when the content of the drug is higher than a certain level, the release rate is increased, so there is a problem in that a sufficient sustained release effect cannot be obtained. The present invention exhibits a sufficient sustained release effect by effectively controlling the release rate of rivastigmine from the beginning while the content of rivastigmine encapsulated in the individual microsphere is high.

The sustained release microsphere for injectable formulations according to the present invention has an average particle size of 10 µm or more, preferably, 15 to 120 µm, or more preferably, 20 to 100 µm. When the average particle size range is shown, an appropriate level may be exhibited without excessively increasing the drug rate, and the convenience of administration may be high. The term "average particle size" or "average particle diameter" used in the present invention is a particle size corresponding 50% of the volume % in the particle size distribution curve, which means the average particle diameter (Median Diameter) and is represented by D50 or D(v, 0.5).

Preferably, the sustained release microsphere for injectable formulations containing rivastigmine of the present invention is characterized by showing an excellent encapsulation efficiency compared to other microspheres prepared using the same content (target loading amount) as rivastigmine during microsphere preparation.

The sustained release microsphere for injectable formulations containing rivastigmine of the present invention is not limited thereto, but may release one or more active ingredients selected from the group consisting of rivastigmine and pharmaceutically acceptable salt thereof for 1 week or more, 2 weeks or more, 1 month or more, 2 months or more, or 3 months or more. Furthermore, the sustained release microsphere for injectable formulations containing rivastigmine of the present invention is not particularly limited in this release pattern, but when administered in vivo, one or more active ingredients selected from the group consisting of rivastigmine and pharmaceutically acceptable salt thereof is preferably released in less than 2% within 1 hour, 15% within 1 day.

The sustained release microsphere for injectable formulations containing rivastigmine according to the present invention is preferably, as it comprises (encapsulates) a high content of rivastigmine in the microsphere, and it do not have a problem of increasing the drug release rate that may occur when an excessive amount of drug is encapsulated in the microsphere. Moreover, the formulation according to the present invention is an injectable formulation, and has relatively few or no side effects such as a gastrointestinal side effects problem caused by oral administration of rivastigmine and skin irritation problem that occurs when used as a percutaneous absorption agent.

Hereinafter, a method for preparation of the sustained release microsphere for injectable formulations containing rivastigmine of the present invention will be described in detail.

The sustained release microsphere for injectable formulations containing rivastigmine according to the present invention may be prepared, for example, using "a solvent extraction and evaporation method", but the preparation method is not limited thereto.

As one embodiment of the method for preparation of a sustained release microsphere for injectable formulations containing rivastigmine according to the present invention, this preparation method comprises (a) dissolving one or more active ingredients selected from the group consisting of rivastigmine and pharmaceutically acceptable poorly soluble salt thereof, and one or more biodegradable polymers in one or more organic solvents to prepare a rivastigmine-polymer solution (dispersed phase), (b) adding the rivastigmine-polymer solution prepared in the step (a) to an aqueous solution phase (continuous phase) containing a surfactant to prepare emulsion, (c) extracting and evaporating the organic solvent from a dispersed phase in the emulsion prepared in the step (b) to the continuous phase to form a microsphere, and (d) recovering the microsphere from the continuous phase of the step (c) to prepare a sustained release microsphere for injectable formulations containing rivastigmine.

For the matters regarding one or more active ingredients selected from the group consisting of rivastigmine and pharmaceutically acceptable salt thereof and one or more biodegradable polymers used in the step (a), the matters defined in the sustained release microsphere for injectable formulations containing rivastigmine may be applied as they are.

In a preferable aspect, the pharmaceutically acceptable poorly soluble salt of rivastigmine may be freeze dried, low pressure dried or hot air dried before preparation of the microsphere. In other words, the pharmaceutically acceptable poorly soluble salt of rivastigmine may be freeze-dried powder, low pressure dried powder or hot air dried powder. More preferably, the pharmaceutically acceptable poorly soluble salt of rivastigmine may be freeze-dried powder. When rivastigmine is not a pharmaceutically acceptable poorly soluble salt, that is, a water-soluble salt such as rivastigmine tartrate or freebase, it is difficult to prepare a dried matter by the drying method including freeze drying, but the pharmaceutically acceptable salt of rivastigmine can be comprised in a microsphere as a dried matter by the drying method to further improve the encapsulation efficiency of the microsphere.

For example, in case of rivastigmine pamoate, it may be exhibited different properties in sticky or crystalline form depending on the molar ratio of rivastigmine and pamoic acid, but in case of showing sticky properties, when a raw material powdered through freeze drying, low pressure drying or hot air drying is used, handling may be made more easily during preparation of the microsphere, but not limited thereto. Freeze drying, low pressure drying or hot air drying methods known in the art may be used without limitation, and conditions such as temperature, pressure and time during drying may be appropriately changed according to the content of the rivastigmine freebase or poorly soluble salt.

In addition, in the step a), in addition to one or more active ingredients selected from the group consisting of rivastigmine and pharmaceutically acceptable poorly soluble salt thereof and one or more biodegradable polymers, fatty acid or pamoic acid may be further dissolved in an organic solvent as a release adjusting agent. The fatty acid used as the release adjusting agent may include one or more selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, isocrotonic acid, oleic acid, elaidic acid, sorbic acid, linoleic acid and arachidonic acid as examples. Regarding the fatty acid or pamoic acid, the matters regarding the microsphere may be applied as they are.

Furthermore, the type of the organic solvent dissolving the active ingredient and biodegradable polymer is not particularly limited, but preferably, one or more solvents selected from the group consisting of dichloromethane, chloroform, ethyl acetate, methyl ethyl ketone, acetone, acetonitrile, dimethyl sulfoxide, dimethyl formamide, N-methyl pyrrolidone, acetic acid, methyl alcohol, ethyl alcohol, propyl alcohol and benzyl alcohol may be used. In one embodiment of the present invention, dichloromethane may be used as a single solvent, or dichloromethane and N-methyl pyrrolidone or dimethyl sulfoxide as a co-solvent thereof may be used to prepare a sustained release microsphere for injectable formulations according to the present invention.

In addition, the active ingredient and the biodegradable polymer may be prepared at a content ratio (active ingredient:biodegradable polymer) of 1:9 to 3:1 based on the weight. Preferably, it may be 1:7 to 2:1, and more preferably, it may be 1:4 to 3:2.

In the step (b), the method for uniformly mixing a continuous phase containing a rivastigmine-polymer solution and a surfactant is not particularly limited, but it may be performed using a high-speed stirrer, an in-line mixer, a membrane emulsion method, a microfluidics emulsion method, and the like. When emulsion is formed using a high-speed stirrer or in-line mixer, it is difficult to obtain uniform emulsion, and therefore, it is preferable to additionally perform a sieving process between the step (c) and step (d). Using a membrane emulsion method or microfluidics emulsion method is more preferable, since a uniform size of emulsion can be obtained and therefore an additional sieving process, and the like are not required between the step (c) and step (d) described below.

The type of the surfactant used in the step (b) is not particularly limited, and any one can be used as long as it can help rivastigmine-polymer solution form a dispersed phase of stable droplets in a continuous phase. The surfactant may be preferably, selected from the group consisting of methyl cellulose, polyvinyl pyrrolidone, carboxymethyl cellulose, lecithin, gelatin, polyvinyl alcohol, polyoxyethylene sorbitan fatty acid ester and polyoxyethylene castor oil derivatives and mixtures thereof, and most preferably, polyvinyl alcohol may be used.

In the step (b), the content of the surfactant in the continuous phase containing the surfactant may be 0.01%(w/v) to 20%(w/v), preferably, 0.1%(w/v) to 5%(w/v), based on the total volume of the continuous phase comprising the surfactant. When the content of the surfactant is 0.01%(w/v) or more, a droplet form of dispersed phase or emulsion may be better formed in the continuous phase, and when the content of the surfactant is 20%(w/v) or less, the surfactant is not included in excess and thus it may be easier to remove the surfactant after a microsphere is formed in the continuous phase.

In addition, as the continuous phase of the step (b), water, or a mixed solvent of water and one or more kinds selected from the group consisting of methyl alcohol, ethyl alcohol, propyl alcohol and ethyl acetate may be used.

In the step (c), when emulsion comprising a dispersed phase in a droplet form and a continuous phase containing a surfactant is maintained or stirred at a temperature less than the boiling point of the organic solvent for a certain time, for example, 2 hours to 48 hours, the organic solvent may be extracted to a continuous phase from the droplet form of rivastigmine-polymer solution as the dispersed phase. A part of the organic solvent extracted in a continuous phase may be evaporated from the surface. As the organic solvent is extracted and evaporated from the rivastigmine-polymer solution in the droplet form, the dispersed phase in the droplet form may be solidified to form a microsphere.

In the step (c), to additionally remove the organic solvent effectively, it is not limited thereto, but heat may be applied to maintain the temperature of the continuous phase at 25°C or higher, preferably 35°C or higher, more preferably at the boiling point of the solvent ±10°C for a certain time.

In the step (d), the method for recovering the sustained release microsphere for injectable formulations of rivastigmine may be performed using various known techniques, and for example, a method such as filtering or centrifugation may be used.

Between the step (c) and step (d), the residual surfactant is removed through filtering and washing and it is filtered again to recover a sustained release microsphere for injectable formulations of rivastigmine.

The washing step to remove the residual surfactant may be commonly performed using water, and the washing step may be repeated several times.

Furthermore, as described above, when emulsion is formed using a high-speed stirrer or in-line mixer, between the step (c) and step (d), uniform microspheres may be obtained by additionally using a sieving process. The sieving process may be performed using known techniques and microspheres in a uniform size may be obtained by filtering out microspheres of small particles and large particles using sieving membranes of different sizes.

In the preparation method of the present invention, after the step (d) or after the filtering and washing step, the obtained microspheres are dried using a common drying method, and thereby, finally, dried microspheres may be obtained.

In other aspect, the present invention provides a long-lasting injectable formulation for preventing or treating Alzheimer's disease comprising the sustained release microsphere(s) for injectable formulations. When formulated into an injectable formulation, the sustained release microsphere for injectable formulations may be formulated in aqueous or oil suspension by addition of an appropriate excipient. For example, when the microsphere(s) is formulated into suspension, those skilled in the art can formulate it by selecting a dispersion medium in which the microsphere can exhibit excellent dispersibility. In addition, the injectable formulation according to the present invention may further comprise a thickener, a stabilizer, a tonicifying agent, a pH adjusting agent, a surfactant, an excipient and/or a carrier. The available tonicifying agent may include an aqueous excipient or saccharide such as mannitol, sucrose, sorbitol, trehalose, lactose, sodium chloride, or the like, and the thickener may include sodium carmellose, sodium carboxymethyl cellulose, povidone, and the like as examples. As the surfactant, as polyoxyethylene sorbitan kinds, polysorbate 80, polysorbate 20, or the like may be used, and as sorbitan ester kinds, span 80, span 20, or the like may be available. In addition, as the buffer, sodium monohydrogenphosphate, citric acid anhydrous, sodium hydroxide, sodium chloride, and the like may be used. In one embodiment, when the microsphere is formulated into an injectable formulation, the microsphere may be present in a vial separate from the dispersion medium, and may be prepared as suspension immediately prior to administration to a patient. When the microsphere is formulated into an injectable formulation, in one embodiment, the present invention provides a kit comprising the sustained release microsphere for injectable formulations, a dispersion medium and a syringe. Alternatively, the sustained release microsphere for injectable formulations and dispersion medium are filled in the syringe, but may be present independently of each other in a separate compartment within the syringe.

### [MODE FOR INVENTION]

### [Example]

Hereinafter, the present invention will be described in more detail by the following examples. However, the following examples illustrate the present invention only, but the contents of the present invention are not limited by the following examples.

### Example 1: Preparation of rivastigmine pamoate

In order to prepare rivastigmine pamoate, pamoic acid (manufacturer: Acros Organics, Belgium) 4.66g was dissolved in dimethyl sulfoxide (manufacturer: Samchun Chemicals, Korea) 100 mL, and rivastigmine (manufacturer: Hwail Pharm, Korea) 10.01 g was added to this solution, and then it was stirred at 50°C for 16 hours and reacted. This was cooled to the room temperature, and then was slowly added to ultrapure water 600 mL and was deposited while stirring for 2 hours. The deposited materials were precipitated or filtered to recover them and were washed with ultrapure water several times. The moisture was removed and it was freeze dried to prepare Sample 1-1.

Samples 1-2, 1-3 and 1-4 were prepared in the same manner as Sample 1-1 except for using the dose of rivastigmine and pamoic acid as shown in the following table.

**[Table 1]**

| Sample | Reaction molar ratio (Rivastigmine:pamoic acid) | Rivastigmine usage (g) | Pamoic acid usage (g) |
|---|---|---|---|
| Sample 1-1 | 1:0.3 | 10.01 | 4.66 |
| Sample 1-2 | 1:0.5 | 10.01 | 7.77 |
| Sample 1-3 | 1:0.7 | 10.01 | 10.87 |
| Sample 1-4 | 1:1 | 10.01 | 15.54 |

As could be confirmed in Table 1 above, it could be confirmed that all of the powdered or crystalline rivastigmine pamoate through a drying method such as freeze drying after preparing rivastigmine pamoate was prepared in an appropriate shape for preparing a microsphere.

### Comparative example 1: Preparation of PLA sustained release microsphere formulation comprising tartrate

A dispersed phase was prepared by mixing a biocompatible polymer, Resomer R 203H (IV=0.25-0.35 dL/g; manufacturer: Evonik, Germany) 3.23 g and rivastigmine tartrate (manufacturer: MSN, India) 1.02g with dichloromethane (manufacturer: J.T.Baker, U.S.) 7.04 g and N-methyl pyrrolidone (manufacturer: JUNSEI, Japan) 9.700 mL. The dispersed phase was sufficiently dissolved by stirring for 30 minutes or more. As a continuous phase, 1.0%(w/v) polyvinyl alcohol (viscosity: 4.8-5.8 mPa·s) aqueous solution was used, and the continuous phase 550 mL was supplied to a membrane emulsifying device and at the same time, the dispersed phase prepared was injected to prepare a microsphere, and the microsphere suspension was placed in a preparation container and stirred at 200 rpm. The temperature of the membrane emulsifying device and preparation container was maintained at 25°C, and when injection of the dispersed phase was completed, it was stirred for 30 minutes, and then the temperature of the microsphere suspension was increased to 45°C and it was maintained for 3 hours and the organic solvent was removed. When the removal of the organic solvent was completed, the temperature of the microsphere suspension was lowered to 25°C. The microsphere suspension was washed with distilled water several times repeatedly, and then it was recovered and dried to prepare Comparative sample 1.

The dispersed phase of Comparative sample 2 was prepared by mixing a biocompatible polymer, Resomer R 203H 3.40 g and rivastigmine tartrate 1.60g with dichloromethane 11.32g and N-methyl pyrrolidone 3.199 mL, and the continuous phase was prepared substantially in the same manner as the Comparative sample 1, except for preparing the continuous phase 1,300 mL with 1.0%(w/v) polyvinyl alcohol aqueous solution.

### Example 2: Preparation of sustained release microsphere formulation comprising rivastigmine

A dispersed phase was prepared by mixing a biocompatible polymer, Resomer R 203H (IV=0.25-0.35 dL/g; manufacturer: Evonik, Germany) 6.68 g and rivastigmine (manufacturer: Hwail Pharm, Korea) 1.18g with dichloromethane (manufacturer: J.T.Baker, U.S.) 16.68 g. The dispersed phase was sufficiently dissolved by stirring for 30 minutes or more. As a continuous phase, 1.0%(w/v) polyvinyl alcohol (viscosity: 4.8-5.8 mPa·s) aqueous solution was used, and the continuous phase 2,502 mL was supplied to a membrane emulsifying device and at the same time, the dispersed phase prepared was injected to prepare a microsphere, and the microsphere suspension was placed in a preparation container and stirred at 200 rpm. The temperature of the membrane emulsifying device and preparation container was maintained at 25°C, and when injection of the dispersed phase was completed, it was stirred for 30 minutes, and then the temperature of the microsphere suspension was increased to 45°C and it was maintained for 3 hours and the organic solvent was removed. When the removal of the organic solvent was completed, the temperature of the microsphere suspension was lowered to 25°C. The microsphere suspension was washed with distilled water several times repeatedly, and then it was recovered and dried to prepare a microsphere of Sample 2-1.

The Sample 2-2 microsphere was prepared substantially in the same manner as the microsphere of Sample 2-1, except for using the dose of the polymer, rivastigmine as an active ingredient, solvent and continuous phase.

For the microsphere of Sample 2-3, as a polymer, a blend of PLA/PLGA was used, and specifically, it was prepared substantially in the same manner as the microsphere of Sample 2-1, except for using biocompatible polymers, Resomer R 205S (IV=0.55-0.75 dL/g; manufacturer: Evonik, Germany) 4.45 g and Resomer RG 753H (IV=0.32-0.44 dL/g; manufacturer: Evonik, Germany) 2.22 g, and using the dose of rivastigmine as an active ingredient, solvent and continuous phase as the table below.

**[Table 2]**

| Sample | Polymer usage (g) | Active ingredient usage (g) | Solvent usage (g) | Continuous phase usage (mL) |
|---|---|---|---|---|
| Sample 2-1 | PLA (6.68) | 1.18 | 16.68 | 2,502 |
| Sample 2-2 | PLA (2.56) | 0.64 | 6.41 | 758 |
| Sample 2-3 | PLA (4.45)/PLGA (2.22) | 2.23 | 33.37 | 5,003 |

### Example 3: Preparation of sustained release microsphere formulation comprising rivastigmine pamoate

A dispersed phase was prepared by mixing Resomer R 203H (IV=0.25-0.35 dL/g; manufacturer: Evonik, Germany) 3.80 g as a biocompatible polymer, PLA, and Sample 1-2 1.20 g according to Example 1 with dichloromethane (manufacturer: J.T.Baker, U.S.) 9.51 g and N-methyl pyrrolidone (manufacturer: JUNSEI, Japan) 2.400 mL. The dispersed phase was sufficiently dissolved by stirring for 30 minutes or more. As a continuous phase, 1.0%(w/v) polyvinyl alcohol (viscosity: 4.8-5.8 mPa·s) aqueous solution was used, and the continuous phase 1,100 mL was prepared in a preparation container and a homogenizer was immersed in the continuous phase and installed. The dispersed phase prepared was injected into the homogenizer and a microsphere was prepared, and the microsphere suspension in the preparation container was stirred at 200 rpm. The temperature of the homogenizer and preparation container was maintained at 25°C, and when the injection of the dispersed phase was completed, it was stirred for 30 minutes, and then the temperature of the microsphere suspension was increased to 45°C and maintained for 3 hours to remove an organic solvent. When the removal of the organic solvent was completed, the temperature of the microsphere suspension was lowered to 25°C. The microsphere suspension was washed with distilled water several times repeatedly and then recovered and dried to prepare a microsphere of Sample 3-1.

As the dispersed phase, a blend of PLA/PLGA was used, and specifically, it was prepared by mixing Resomer R 205S (IV=0.55-0.75 dL/g; manufacturer: Evonik, Germany) 4.12 g and Resomer RG 753H (IV=0.32-0.44 dL/g; manufacturer: Evonik, Germany) 2.06 g, and Sample 1-4 3.83 g according to Example 1 with dichloromethane (manufacturer: J.T.Baker, U.S.) 21.70 g and dimethyl sulfoxide (manufacturer: Samchun Chemicals, Korea) 8.418 mL. The dispersed phase was sufficiently dissolved by stirring for 30 minutes or more. As a continuous phase, 1.0%(w/v) polyvinyl alcohol (viscosity: 4.8-5.8 mPa·s) aqueous solution was used, and the continuous phase 4,630 mL was supplied to a membrane emulsifying device and at the same time, the dispersed phase prepared was injected to prepare a microsphere, and the microsphere suspension was placed in a preparation container and stirred at 200 rpm. The temperature of the membrane emulsifying device and preparation container was maintained at 25°C, and when injection of the dispersed phase was completed, it was stirred for 30 minutes, and then the temperature of the microsphere suspension was increased to 45°C and it was maintained for 3 hours and the organic solvent was removed. When the removal of the organic solvent was completed, the temperature of the microsphere suspension was lowered to 25°C. The microsphere suspension was washed with distilled water several times repeatedly, and then it was recovered and dried to prepare a microsphere of Sample 3-2.

For the microsphere of Sample 3-3, as a polymer, a blend of PLA/PLGA was used, and specifically, it was prepared substantially in the same manner as the microsphere of Sample 3-2, except for using polymers, Resomer R 205S (IV=0.55-0.75 dL/g; manufacturer: Evonik, Germany) 4.40 g and Resomer RG 753H (IV=0.32-0.44 dL/g; manufacturer: Evonik, Germany) 2.20 g, and using the dose of Sample 1-2 according to Example 1 as an active ingredient, solvent and continuous phase as the table below.

For the microsphere of Sample 3-4, as a polymer, a blend of PLA/PLGA was used, and specifically, it was prepared substantially in the same manner as the microsphere of Sample 3-2, except for using Resomer R 205S (IV=0.55-0.75 dL/g; manufacturer: Evonik, Germany) 3.81 g and Resomer RG 753H (IV=0.32-0.44 dL/g; manufacturer: Evonik, Germany) 1.91 g, and using the dose of Sample 1-3 according to Example 1, solvent and continuous phase as an active ingredient as the table below.

The microsphere of Sample 3-5 was prepared substantially in the same manner as the microsphere of Sample 3-2, except for using Resomer RG 858S (IV=1.3-1.7 dL/g; manufacturer: Evonik, Germany) as a PLGA polymer, and using the dose of Sample 1-4 according to Example 1, solvent and continuous phase as the table below.

The microsphere of Sample 3-6 was prepared substantially in the same manner as the microsphere of Sample 3-2, except for using Resomer R 203H (IV=0.25-0.35 dL/g; manufacturer: Evonik, Germany) and Resomer R 205S (IV=0.55-0.75 dL/g; manufacturer: Evonik, Germany) as a PLA polymer, and using the dose of Sample 1-2 according to Example 1, solvent and continuous phase as the table below, and using a membrane emulsifying device instead of a homogenizer.

The microsphere of Sample 3-7 was prepared substantially in the same manner as the microsphere of Sample 3-2, except for using Resomer RG 503H (IV=0.32-0.44 dL/g; manufacturer: Evonik, Germany) as a PLGA polymer, and using the dose of Sample 1-2 according to Example 1, solvent and continuous phase as the table below.

The microsphere of Sample 3-8 was prepared substantially in the same manner as the microsphere of Sample 3-2, except for using Resomer RG 653H (IV=0.32-0.44 dL/g; manufacturer: Evonik, Germany) as a PLGA polymer, and using the dose of Sample 1-2 according to Example 1, solvent and continuous phase as the table below.

The microsphere of Sample 3-9 was prepared substantially in the same manner as the microsphere of Sample 3-2, except for using Resomer RG 753H (IV=0.32-0.44 dL/g; manufacturer: Evonik, Germany) as a PLGA polymer, and using the dose of Sample 1-2 according to Example 1, solvent and continuous phase as the table below.

The microsphere of Sample 3-10 was prepared substantially in the same manner as the microsphere of Sample 3-2, except for using Resomer R 203H (IV=0.25-0.35 dL/g; manufacturer: Evonik, Germany) as a PLA polymer, and using the dose of Sample 1-2 according to Example 1, solvent and continuous phase as the table below.

For the microsphere of Sample 3-11, as a polymer, a blend of PLA/PLGA was used, and specifically, it was prepared substantially in the same manner as the microsphere of Sample 3-2, except for using Resomer R 205S (IV=0.55-0.75 dL/g; manufacturer: Evonik, Germany) and Resomer RG 858S (IV=1.3-1.7 dL/g; manufacturer: Evonik, Germany), and using the dose of Sample 1-2 according to Example 1, solvent and continuous phase.

The microsphere of Sample 3-12 was prepared substantially in the same manner as the microsphere of Sample 3-2, except for using Resomer RG 858S (IV=1.3-1.7 dL/g; manufacturer: Evonik, Germany) as a PLGA polymer, and using the dose of Sample 1-2 according to Example 1, solvent and continuous phase as the table below.

The microsphere of Sample 3-13 was prepared substantially in the same manner as the microsphere of Sample 3-2, except for using Resomer RG 858S (IV=1.3-1.7 dL/g; manufacturer: Evonik, Germany) as a PLGA polymer, and using the dose of Sample 1-2 according to Example 1, solvent and continuous phase as the table below.

**[Table 3]**

| Sample | Polymer usage (g) | Active ingredient usage (g) | Solvent usage | Continuous phase usage (mL) |
|---|---|---|---|---|
| Sample 3-1 | PLA (3.80) | 1.20 | dichloromethane (9.51 g)/ N-methyl pyrrolidone (2.400 mL) | 1,100 |
| Sample 3-2 | PLA (4.12)/PLGA (2.06) | 3.83 | dichloromethane (21.70 g)/ dimethyl sulfoxide (8.418 mL) | 4,630 |
| Sample 3-3 | PLA (4.40)/PLGA (2.20) | 2.40 | dichloromethane (33.01 g) | 4,952 |
| Sample 3-4 | PLA (3.81)/PLGA (1.91) | 3.28 | dichloromethane (27.15 g)/ dimethyl sulfoxide (3.318 mL) | 4,172 |
| Sample 3-5 | PLGA (1.98) | 1.23 | dichloromethane (13.32 g)/dimethyl sulfoxide (4.082 mL) | 1,975 |
| Sample 3-6 | PLA R 203H (1.61)/PLA R 205S (4.84) | 3.55 | dichloromethane (32.47 g) | 4,836 |
| Sample 3-7 | PLGA (6.45) | 3.55 | dichloromethane (33.07 g) | 4,836 |
| Sample 3-8 | PLGA (6.45) | 3.55 | dichloromethane (32.29 g) | 4,836 |
| Sample 3-9 | PLGA (6.45) | 3.55 | dichloromethane (32.26 g) | 4,836 |
| Sample 3-10 | PLA (5.56) | 4.44 | dichloromethane (27.85 g) | 4,171 |
| Sample 3-11 | PLA (2.64)/PLGA (2.64) | 4.22 | dichloromethane (31.09 g) | 4,661 |
| Sample 3-12 | PLGA (1.17) | 0.43 | dichloromethane (9.02 g)/dimethyl sulfoxide (1.417 mL) | 1,351 |
| Sample 3-13 | PLGA (3.50) | 4.00 | dichloromethane (31.86 g) | 4,779 |

### Example 4: Preparation of PLA sustained release microsphere formulation comprising rivastigmine and fatty acid

A dispersed phase was prepared by mixing a biocompatible polymer, Resomer R 203H (IV=0.25-0.35 dL/g; manufacturer: Evonik, Germany) 3.31 g and rivastigmine (manufacturer: Hwail Pharm, Korea) 1.00 g and capric acid (manufacturer: Alfa Aesar, U.S.) 0.69 g with dichloromethane (manufacturer: J.T.Baker, U.S.) 8.27 g. The dispersed phase was sufficiently dissolved by stirring for 30 minutes or more. As a continuous phase, 2.0%(w/v) polyvinyl alcohol (viscosity: 4.8-5.8 mPa·s) aqueous solution was used, and the continuous phase 950 mL was supplied to a membrane emulsifying device and at the same time, the dispersed phase prepared was injected to prepare a microsphere, and the microsphere suspension was placed in a preparation container and stirred at 200 rpm.

The temperature of the membrane emulsifying device and preparation container was maintained at 25°C, and when injection of the dispersed phase was completed, it was stirred for 30 minutes, and then the temperature of the microsphere suspension was increased to 35°C and it was maintained for 3 hours and the organic solvent was removed. When the removal of the organic solvent was completed, the temperature of the microsphere suspension was lowered to 25°C. The microsphere suspension was washed with distilled water several times repeatedly, and then it was recovered and dried to prepare a microsphere of Sample 4-1.

Except for using lauric acid for Sample 4-2 microsphere, myristic acid for Sample 4-3 microsphere, palmitic acid for Sample 4-4 microsphere and stearic acid for Sample 4-5 microsphere, respectively, as fatty acid, and using the dose of other polymers, rivastigmine as an active ingredient, solvent and continuous phase as Table 4 below.

**[Table 4]**

| Sample | Polymer usage (g) | Active ingredient usage (g) | Solvent usage (g) | Fatty acid usage (g) | Continuous phase usage (mL) |
|---|---|---|---|---|---|
| Sample4-1 | PLA (3.31) | 1.00 | dichloromethane (8.27) | Capric acid (0.69) | 950 |
| Sample4-2 | PLA (3.20) | 1.00 | dichloromethane (8.00) | Lauric acid (0.80) | 900 |
| Sample4-3 | PLA (3.09) | 1.00 | dichloromethane (7.72) | Myristic acid (0.91) | 900 |
| Sample4-4 | PLA (3.50) | 1.00 | dichloromethane (8.75) | Palmitic acid (0.50) | 1,000 |
| Sample4-5 | PLA (3.43) | 1.00 | dichloromethane (8.58) | Stearic acid (0.57) | 1,000 |

### Example 5: Preparation of sustained release microsphere formulation comprising rivastigmine and rivastigmine pamoate

A dispersed phase was prepared by mixing a biocompatible polymer, Resomer RG 858S (IV=1.3-1.7 dL/g; manufacturer: Evonik, Germany) 3.61 g and rivastigmine (manufacturer: Hwail Pharm, Korea) 0.76 g and Sample 1-2 0.89 g with dichloromethane (manufacturer: J.T.Baker, U.S.) 32.82 g and dimethyl sulfoxide(manufacturer: Samchun Chemicals, Korea) 1.850 mL. The dispersed phase was sufficiently dissolved by stirring for 30 minutes or more. As a continuous phase, 1.0%(w/v) polyvinyl alcohol (viscosity: 4.8-5.8 mPa·s) aqueous solution was used, and the continuous phase 4,923 mL was supplied to a membrane emulsifying device and at the same time, the dispersed phase prepared was injected to prepare a microsphere, and the microsphere suspension was placed in a preparation container and stirred at 200 rpm.

The temperature of the membrane emulsifying device and preparation container was maintained at 25°C, and when injection of the dispersed phase was completed, the temperature of the microsphere suspension was maintained as 45°C for 3 hours and the organic solvent was removed. When the removal of the organic solvent was completed, the temperature of the microsphere suspension was lowered to 25°C.

The microsphere suspension was washed with ultrapure water several times repeatedly to remove residual polyvinyl alcohol, and the microsphere was freeze dried to prepare a microsphere of Sample 5.

### Experimental example 1: Measurement of rivastigmine and pamoate contents in rivastigmine pamoate

In order to measure the contents of rivastigmine and pamoate of rivastigmine pamoate prepared in Example 1, the sample 10 mg was completely dissolved with dimethyl sulfoxide, and then was diluted into a mobile phase. The diluted solution 20 uL was injected to HPLC and was measured at a detection wavelength 210 nm. The column used in the present measurement was Inertsil ODS-3, 5 um, 4.6x250 mm, and the mobile phase was used by mixing phosphate buffer solution (pH 6.0) and acetonitrile at a ratio of 65:35(v/v). The measured contents were shown in Table 5.

**[Table 5]**

| Comparison of drug content of rivastigmine pamoate | | |
|---|---|---|
| Sample | Content analysis result | |
| | Rivastigmine (% by weight) | Pamoate (% by weight) |
| Sample 1-1 | 57.47 | 40.49 |
| Sample 1-2 | 52.45 | 40.47 |
| Sample 1-3 | 41.10 | 53.79 |
| Sample 1-4 | 32.19 | 68.93 |

### Experimental example 2: Measurement of rivastigmine content in microsphere according to drug type

In order to measure the rivastigmine content in the microsphere prepared in Example and Comparative example above, the microsphere 10 mg was completely dissolved with dimethyl sulfoxide, and then was diluted into a mobile phase. The diluted solution 20 uL was injected to HPLC and was measured at a detection wavelength 210 nm. The column used in the present measurement was Inertsil ODS-3, 5 um, 4.6x250 mm, and the mobile phase was used by mixing phosphate buffer solution (pH 6.0) and acetonitrile at a ratio of 65:35(v/v). The measured encapsulation efficiencies were shown in Table 6.

**[Table 6]**

| Comparison of drug content and encapsulation efficiency according to drug kind and drug input amount | | | | |
|---|---|---|---|---|
| Classification | Raw material | Content analysis result | | |
| | | TL (%) | Riv (%) | E.E. (%) |
| Comparative sample 1 | Rivastigmine tartrate | 15 | 3.12 | 21 |
| Sample 2-1 | Rivastigmine freebase | 15 | 4.61 | 31 |
| Sample 3-3 | Rivastigmine pamoate | 15 | 10.28 | 69 |
| Sample 3-4 | Rivastigmine pamoate | 15 | 11.52 | 77 |
| Sample 3-5 | Rivastigmine pamoate | 15 | 14.27 | 95 |
| Comparative sample 2 | Rivastigmine tartrate | 20 | 6.98 | 35 |
| Sample 2-2 | Rivastigmine freebase | 20 | 8.43 | 42 |
| Sample 2-3 | Rivastigmine freebase | 25 | 8.62 | 34 |
| Sample 3-6 | Rivastigmine pamoate | 20 | 14.69 | 73 |
| Sample 3-7 | Rivastigmine pamoate | 20 | 16.75 | 84 |
| Sample 3-8 | Rivastigmine pamoate | 20 | 16.07 | 80 |
| Sample 3-9 | Rivastigmine pamoate | 20 | 15.46 | 77 |
| Sample 3-10 | Rivastigmine pamoate | 25 | 19.62 | 78 |
| Sample 3-11 | Rivastigmine pamoate | 25 | 22.26 | 89 |
| Sample 3-12 | Rivastigmine pamoate | 15 | 10.30 | 69 |
| Sample 3-13 | Rivastigmine pamoate | 30 | 26.89 | 90 |

(TL(target loading)(%): Rivastigmine target loading amount during microsphere preparation, Riv (%): Rivastigmine encapsulation efficiency in the actual microsphere, E.E.(Encapsulation efficiency)(%): Encapsulation efficiency of rivastigmine)

According to the result of Table 6 above, it could be confirmed that the sustained release microsphere for injectable formulations comprising rivastigmine pamoate according to the present invention showed excellent rivastigmine encapsulation efficiency compared to the microsphere prepared using the same target loading amount of rivastigmine tartrate and rivastigmine freebase, and it could be confirmed that this was same in the case of different kinds of biodegradable polymers.

### Experimental example 3: Microsphere morphological analysis through an electron microscope

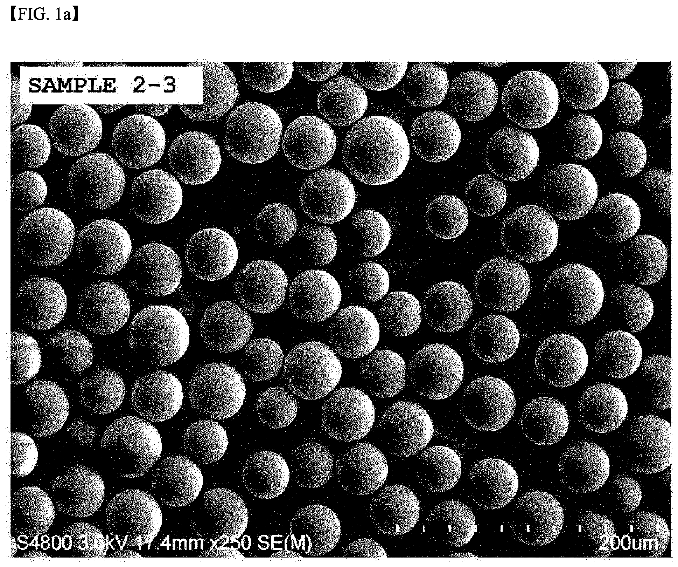

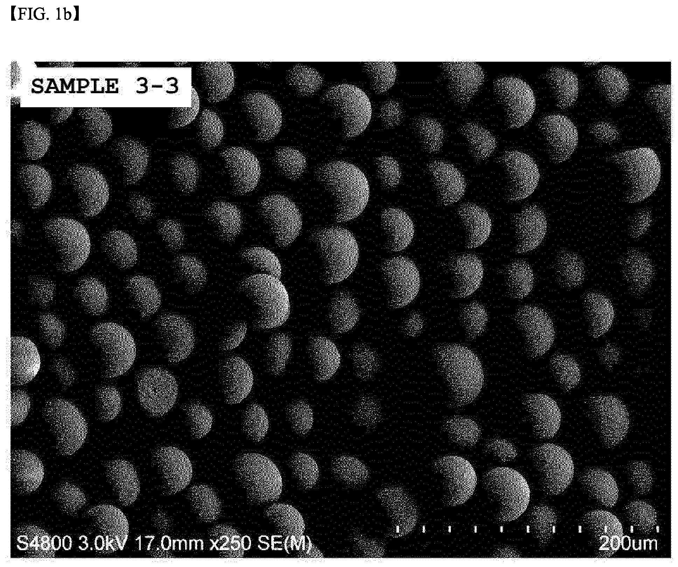

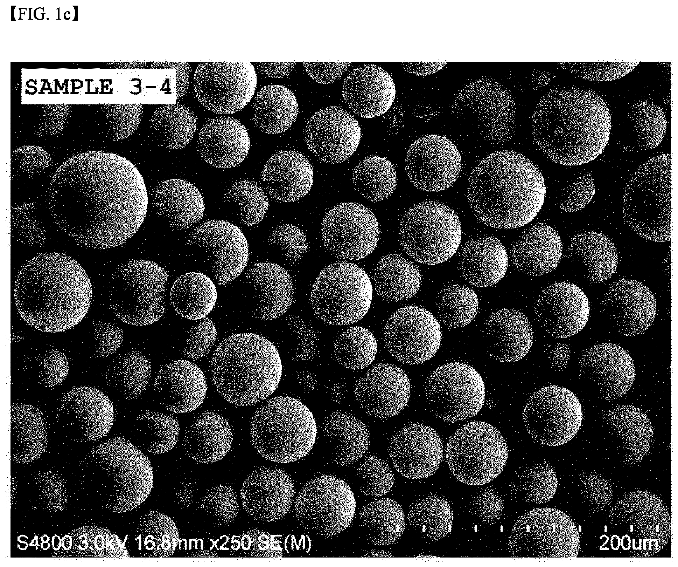

In order to analyze morphological properties of the microspheres prepared in Samples 3-3, 3-4 and Sample 2-3, scanning electron microscope observation was conducted. After fixing the microspheres in a holder with carbon tape, the surface was coated with platinum using a metal coating machine (Cressington, 208HR, UK). The holder was mounted on a scanning electron microscope (Hitachi, S4800, Japan) and the morphological properties of the microspheres were observed with an acceleration voltage of 3.0 kV. The result was shown in FIGs. 1a to 1c.

As could be confirmed in FIGs. 1a to 1c, it could be confirmed that the particle sizes of the microsphere comprising rivastigmine freebase and a biodegradable polymer and microspheres comprising rivastigmine pamoate of Samples 3-3 and 3-4 were all uniform.

### Experimental example 4: Measurement of in vitro drug release rate of microsphere according to drug type

In order to measure the *in vitro* release rate of rivastigmine in the microspheres prepared in each Example and Comparative example above, the following experiment was performed. The microsphere 10 mg was put in an HDPE wide-mouth bottle, and release test solution (pH 7.4) 10 mL was filled and then it was stored in a 37°C incubator. For a sample solution, the supernatant 0.2 mL was taken and secured, and a new release test solution 0.2 mL was added to the wide-mouth bottle. The sample was collected at a set time and the content and release rate were analyzed using HPLC under the same analytical conditions as Experimental example 1.

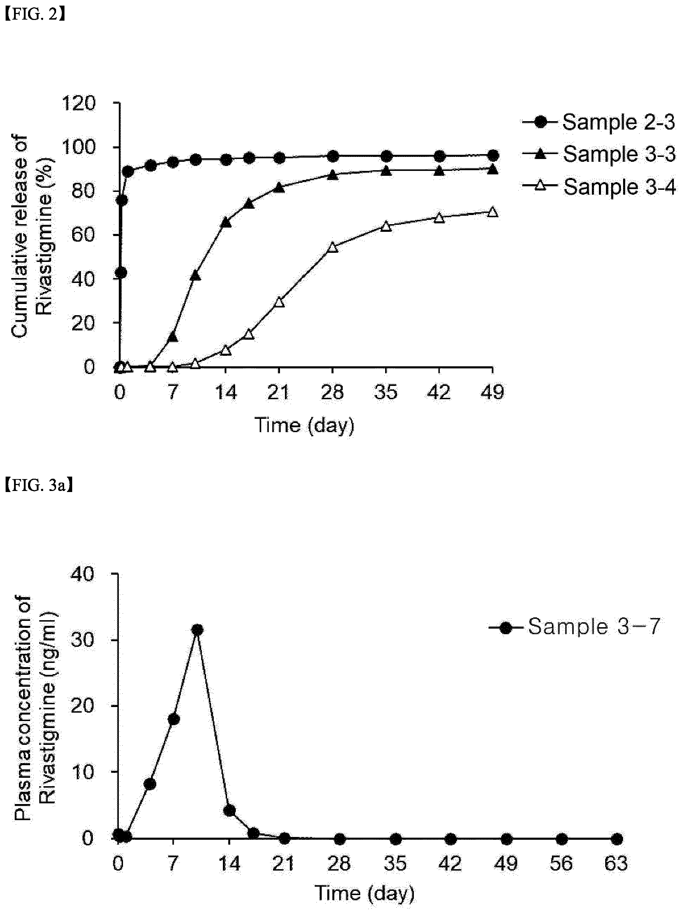

The result was shown in Table 7 and FIG. 2.

**[Table 7]**

| Classification | Cumulative release rate of drug (%) | | | | | | | | |
|---|---|---|---|---|---|---|---|---|---|
| | Day 0 | Day 0.04 | Day 0.13 | Day 0.25 | Day 1 | Day 4 | Day 7 | Day 10 | Day 14 |
| Sample 2-3 | 0 | 0.40 | 43.04 | 76.11 | 89.23 | 91.67 | 93.33 | 94.32 | 94.65 |
| Sample 3-3 | 0 | 0.15 | 0.15 | 0.19 | 0.19 | 0.52 | 14.15 | 42.14 | 65.98 |
| Sample 3-4 | 0 | 0.14 | 0.16 | 0.16 | 0.18 | 0.19 | 0.19 | 1.61 | 7.95 |

| Classification | Cumulative release rate of drug (%) | | | | | | | | |
|---|---|---|---|---|---|---|---|---|---|
| | Day 17 | Day 21 | Day 28 | Day 35 | Day 42 | Day 49 | | | |
| Sample 2-3 | 95.08 | 95.26 | 96.00 | 96.00 | 96.21 | 96.28 | | | |
| Sample 3-3 | 74.49 | 81.89 | 87.44 | 89.53 | 89.53 | 90.31 | | | |
| Sample 3-4 | 15.12 | 29.75 | 54.55 | 64.18 | 68.14 | 70.88 | | | |

As could be confirmed in Table 7 and FIG. 2 above, the microsphere of Sample 2-3 comprising rivastigmine freebase showed a release rate of 40% or more of rivastigmine at 3 hours (0.13 days) after the release test, and showed a release rate close to 90% at the time point of Day 1, and therefore, it was confirmed that the drug was rapidly released in the early stage, and most of the drug was released at the time point of Day 7, while the microspheres of Samples 3-3 and 3-4 comprising rivastigmine pamoate had a well-controlled release rate without rapid drug release until the time point of Day 7 after the release test, and the drug was slowly released over a long period of 49 days, and therefore, it could be confirmed that it was more preferable in terms of drug release to comprise rivastigmine pamoate.

### Experimental example 5: Measurement of content in microsphere according to additive (by fatty acid carbon chain length

In order to measure the content of rivastigmine of the microspheres prepared in Samples 4-1, 4-2, 4-3, 4-4 and 4-5 and Sample 2-2 above, the microspheres 10 mg were completely dissolved with DMSO, and then were diluted into a mobile phase. The diluted solution 20 uL was injected to HPLC and was measured at a detection wavelength 271 nm. The column used in the present measurement was Inertsil ODS-3, 5 um, 4.6x150 mm, and the mobile phase was used by mixing phosphate buffer solution (pH 5.0) and acetonitrile at a ratio (v/v) of 6:4. The measured encapsulation efficiency was shown in Table 8.

**[Table 8]**

| Comparison of drug content and encapsulation efficiency in microsphere according to additive | | | | |
|---|---|---|---|---|
| Classification | Raw material | Content analysis result | | |
| | | TL (%) | Riv (%) | E.E. (%) |
| Sample 2-2 | Rivastigmine freebase | 20 | 8.43 | 42 |
| Sample 4-1 | Rivastigmine freebase + C10:0 fattv acid | 20 | 9.70 | 49 |
| Sample 4-2 | Rivastigmine freebase + C12:0 fattv acid | 20 | 14.01 | 70 |
| Sample 4-3 | Rivastigmine freebase + C14:0 fattv acid | 20 | 15.26 | 76 |
| Sample 4-4 | Rivastigmine freebase + C16:0 fattv acid | 20 | 11.22 | 56 |
| Sample 4-5 | Rivastigmine freebase + C18:0 fattv acid | 20 | 12.33 | 62 |

(TL (%): Rivastigmine target loading amount during microsphere preparation, Riv (%): Rivastigmine encapsulation efficiency in the actual microsphere, E.E.(Encapsulation efficiency)(%): Encapsulation efficiency of rivastigmine, C10:0 fatty acid: capric acid, C12:0 fatty acid: lauric acid, C14:0 fatty acid: myristic acid, C16:0 fatty acid: palmitic acid, and C18 fatty acid: stearic acid)

According to the result of Table 8 above, it could be confirmed that the microsphere further comprising fatty acid as an additive together with rivastigmine showed much more excellent rivastigmine encapsulation efficiency compared to the microsphere not comprising fatty acid.

### Experimental example 6: Analysis of microsphere particle size using laser diffraction method

In order to quantitatively measure the average particle size, distribution and uniformity of the prepared microspheres, analysis of the particle size of the microspheres was conducted using a laser diffraction method. The microspheres 100 mg prepared in Samples 2-1, 3-1, 3-2, 3-3, 3-4, 3-5, 3-6, 3-9, 3-11, 3-12 and 4-1, and Comparative samples 1 and 2 were mixed with 9%(w/v) Tween 20 aqueous solution 1 mL and pipetted to distribute them. The prepared microsphere dispersion was put in a particle size analyzer (CILAS, 990L, France) and it was measured for 10 seconds.

The result was shown in Table 9 below.

**[Table 9]**

| Result of particle size analysis of prepared microspheres (average particle size: 10-120 µm) | |
|---|---|
| Classification | D₅₀ (µm) |
| Comparative sample 1 | 71.74 |
| Comparative sample 2 | 100.54 |
| Sample 2-1 | 54.62 |
| Sample 3-1 | 119.50 |
| Sample 3-2 | 137.94 |
| Sample 3-3 | 45.42 |
| Sample 3-4 | 54.67 |
| Sample 3-5 | 64.87 |
| Sample 3-6 | 41.25 |
| Sample 3-9 | 41.07 |
| Sample 3-11 | 52.88 |
| Sample 3-12 | 106.87 |
| Sample 4-1 | 53.57 |

As could be confirmed in Table 9 above, it could be confirmed that the microspheres comprising rivastigmine pamoate had an average particle size of 150 µm or less.

### Experimental example 7: Single intramuscular administration pharmacokinetics test using Sprague-Dawley rats

In order to evaluate the possibility of the sustained release microsphere containing rivastigmine according to the present invention as a sustained release therapeutic agent, the rivastigmine concentration in rat blood was measured b the following method. The microspheres used for the experiment were microspheres prepared in Samples 3-7, 3-8 and 3-9.

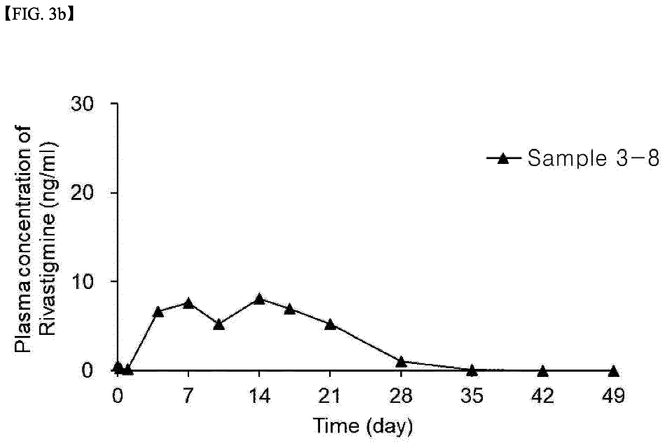

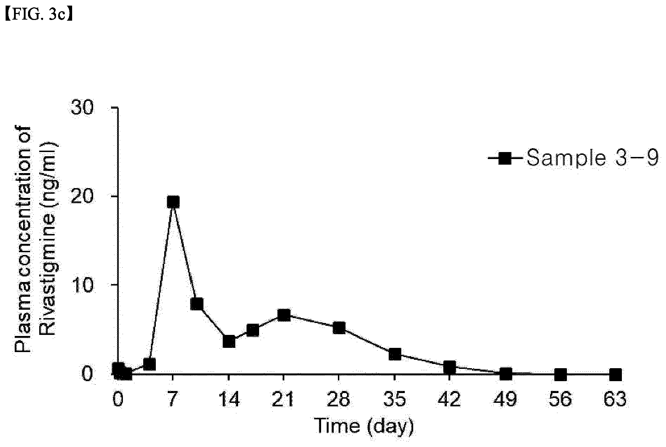

The microspheres were measured so that the rivastigmine dose was 57.9 mg/kg and they were dispersed in 0.350 mL suspension and then intramuscularly injected into SD rats. At predetermined times, 0.25-0.5 mL blood was collected and the concentration of rivastigmine in blood was measured using HPLC, and the result was shown in Table 10 and FIGs. 3a, 3b and 3c. As could be confirmed in Table 10 and FIG. 3, it could be confirmed that the rivastigmine microspheres according to the present invention exhibited an excellent sustained release effect by continuously releasing drug for as short as 14 days and as long as 56 days without an initial burst after administration.

**[Table 10]**

| Cumulative release rate of drug according to elapsed days after administration (%) | | | | | | | | |
|---|---|---|---|---|---|---|---|---|
| Classification | Cumulative release rate of drug according to elapsed days after administration (%) | | | | | | | |
| | Day 0 | Day 0.04 | Day 0.25 | Day 1 | Day 4 | Day 7 | Day 10 | Day 14 |
| Sample 3-7 | 0 | 0.01 | 0.06 | 0.19 | 6.37 | 25.23 | 60.85 | 95.26 |
| Sample 3-8 | 0 | 0.01 | 0.07 | 0.18 | 7.02 | 21.28 | 34.07 | 51.76 |
| Sample 3-9 | 0 | 0.01 | 0.05 | 0.10 | 0.96 | 15.26 | 34.24 | 45.02 |

| Classification | Cumulative release rate of drug according to elapsed days after administration (%) | | | | | | | |
|---|---|---|---|---|---|---|---|---|
| | Day 17 | Day 21 | Dav 28 | Dav 35 | Day 42 | Day 49 | Dav 56 | Dav 63 |
| Sample 3-7 | 99.01 | 99.91 | 100.00 | 100.00 | 100.00 | 100.00 | 100.00 | 100.00 |
| Sample 3-8 | 66.77 | 82.90 | 97.33 | 99.84 | 100.00 | 100.00 | 100.00 | 100.00 |
| Sample 3-9 | 51.04 | 61.81 | 81.13 | 93.27 | 98.35 | 99.86 | 100.00 | 100.00 |

## Claims

1. A sustained release microsphere for injectable formulations comprising a poorly soluble active ingredient and a biodegradable polymer, wherein the active ingredient is at least one selected from the group consisting of rivastigmine and a pharmaceutically acceptable poorly soluble salt thereof, and an amount of the active ingredient is comprised at 7 % by weight or more as rivastigmine based on the total microsphere weight.

2. The sustained release microsphere for injectable formulations according to claim 1, wherein the pharmaceutically acceptable poorly soluble salt of rivastigmine is rivastigmine pamoate.

3. The sustained release microsphere for injectable formulations according to claim 2, wherein the molar ratio of rivastigmine:pamoic acid in the rivastigmine pamoate is 1:0.3 to 1:1.

4. The sustained release microsphere for injectable formulations according to claim 1, further comprising pamoic acid or fatty acid as a release adjusting agent.

5. The sustained release microsphere for injectable formulations according to claim 1, wherein the content of the organic acid contained in the poorly soluble salt of rivastigmine is 2.0 to 50 % by weight based on the total microsphere weight.

6. The sustained release microsphere for injectable formulations according to claim 4, wherein the poorly soluble salt of rivastigmine is comprised as an active ingredient, and the sum of the organic acid content in the poorly soluble salt of rivastigmine and the content of pamoic acid or fatty acid as the release adjusting agent is 2.0 to 50 % by weight based on the total microsphere weight.

7. The sustained release microsphere for injectable formulations according to claim 4, wherein rivastigmine is comprised as an active ingredient, and the sum of the content of pamoic acid or fatty acid as the release adjusting agent is 2.0 to 50 % by weight based on the total microsphere weight.

8. The sustained release microsphere for injectable formulations according to claim 4, wherein the fatty acid is one or more selected from the group consisting of butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, isocrotonic acid, oleic acid, elaidic acid, sorbic acid, linoleic acid and arachidonic acid.

9. The sustained release microsphere for injectable formulations according to claim 1, wherein the biodegradable polymer is a biodegradable polymer with intrinsic viscosity of 0.16 to 1.9 dL/g.

10. The sustained release microsphere for injectable formulations according to claim 1, wherein the biodegradable polymer is at least one selected from the group consisting of poly(lactide-co-glycolide), poly(lactide-co-glycolide)glucose, polylactide, polyglycolide, polycaprolactone or mixture thereof; polyglycolide, polylactide and a copolymer of polyglycolide and polylactide.

11. The sustained release microsphere for injectable formulations according to claim 10, wherein the molar ratio of lactide to glycolide of the copolymer of polyglycolide and polylactide is 40:60 to 90:10.

12. The sustained release microsphere for injectable formulations according to claim 1, wherein the poorly soluble salt of rivastigmine is freeze-dried powder, low pressure dried powder or hot air dried powder.

13. The sustained release microsphere for injectable formulations according to claim 1, wherein the weight-average molecular weight of the biodegradable polymer is 4,000 to 240,000.

14. The sustained release microsphere for injectable formulations according to claim 1, wherein the average particle size of the microsphere is 10µm or more.

15. A method for preparation of the sustained release microsphere for injectable formulations according to any one of claim 1 to claim 14, comprising
(a) dissolving one or more active ingredients selected from the group consisting of rivastigmine and pharmaceutically acceptable poorly soluble salt thereof, and one or more biodegradable polymers in one or more organic solvents to prepare a rivastigmine-polymer solution (dispersed phase);
(b) adding the rivastigmine-polymer solution prepared in the step (a) to an aqueous solution phase (continuous phase) containing a surfactant to prepare emulsion;
(c) extracting and evaporating the organic solvent from the dispersed phase in the emulsion prepared in the step (b) to the continuous phase to form a microsphere; and
(d) recovering the microsphere from the continuous phase of the step (c) to prepare a sustained release microsphere for injectable formulations containing rivastigmine.

16. The method for preparation according to claim 15, wherein the weight ratio of the active ingredient and the biodegradable polymer (active ingredient:biodegradable polymer) is 1:9 to 3:1.

17. The method for preparation according to claim 15, wherein the pharmaceutically acceptable salt of rivastigmine used in the step (a) is freeze-drying, low pressure drying or hot air drying.

18. The method for preparation according to claim 15, wherein fatty acid or pamoic acid is further dissolved in the organic acid as a release adjusting agent in the step a).

19. The method for preparation according to claim 15, wherein the organic solvent is one or more solvents selected from the group consisting of dichloromethane, chloroform, ethyl acetate, methyl ethyl ketone, acetone, acetonitrile, dimethyl sulfoxide, dimethyl formamide, N-methyl pyrrolidone, acetic acid, methyl alcohol, ethyl alcohol, propyl alcohol and benzyl alcohol.

20. The method for preparation according to claim 15, further comprising a sieving process between the step (c) and the step (d).

21. The method for preparation according to claim 15, wherein the surfactant of the step (b) is one or more selected from the group consisting of methyl cellulose, polyvinyl pyrrolidone, carboxymethyl cellulose, lecithin, gelatin, polyvinyl alcohol, polyoxyethylene sorbitan fatty acid ester and polyoxyethylene castor oil derivatives and mixtures thereof.

22. The method for preparation according to claim 15, wherein the continuous phase of the step (b) is water; or a mixed solvent of water and one or more selected from the group consisting of methyl alcohol, ethyl alcohol, propyl alcohol and ethyl acetate.

23. An injectable formulation for preventing or treating Alzheimer's disease, comprising the microsphere according to any one of claim 1 to claim 14.
